# EUROPEAN PATENT SPECIFICATION

(11) **EP 2 100 623 B1**
(45) Date of publication and mention of the grant of the patent: **09.03.2011**
(21) Application number: 09001132.1
(22) Date of filing: 28.01.2009
(51) Int. Cl.: A61L 2/18, A61L 2/03

(54) **Spray dispenser**
Sprühdose
Pulvérisateur

(30) Priority: 26.02.2008 GB 0803439
(43) Date of publication of application: 16.09.2009
(73) Proprietor: Dyson Technology Limited, Malmesbury, Wiltshire SN16 0RP (GB)
(72) Inventor: Simmonds, Kevin John, Malmesbury, Wiltshire, SN16 0RP (GB); Cookson, Matthew, Malmesbury, Wiltshire, SN16 0RP (GB); Lee, Damian Henri, Malmesbury, Wiltshire, SN16 0RP (GB); Brown, Nathan Charles, Malmesbury, Wiltshire, SN16 0RP (GB)
(74) Representative: Booth, Andrew Steven

(56) References cited:
- EP-A- 1 469 102
- EP-A- 1 741 676
- WO-A-02/48054
- JP-A- 2004 129 954
- US-A1- 2004 211 676
- AGLADZE G R ET AL: "Comparative study of hydrogen peroxide electro-generation on gas-diffusion electrodes in undivided and membrane cells" JOURNAL OF APPLIED ELECTROCHEMISTRY, KLUWER ACADEMIC PUBLISHERS, DO, vol. 37, no. 3, 20 January 2007 (2007-01-20), pages 375-383, XP019480551 ISSN: 1572-8838

## Description

The invention relates to a spray dispenser. Particularly, but not exclusively, the invention relates to a hand-held spray dispenser for spraying a liquid on to a surface.

Conventional hand-held spray dispensers comprise a container for the liquid to be sprayed, for example an aqueous solution, and a spray head detachably mounted on the container for spraying the solution from the container. The container is usually in the form of a bottle having a spout to which the spray head is attached. The spray head usually comprises a nozzle, a flexible dip tube extending into the container and a trigger-actuated pump. When the trigger is depressed, the pump forces solution out from the spray head through the nozzle, whilst when the trigger is released, the pump causes solution to be drawn up the tube and into the spray head.

A common use of hand-held dispensers is to spray a sterilising solution on to a surface to kill bacteria. WO02/48054 describes a device which ozonates water for use in sterilising work surfaces or food. The device includes a spray dispenser having a container which the user fills with water and locates on a base station. The base station contains a water softener, an electrolytic cell and a pump for pumping water from the dispenser to the water softener and then to the cell, which generates ozone from the softened water. Under the pumping action of the pump, the ozonated water is returned to the dispenser, together with gaseous O₂ and O₃ generated within the cell. The dispenser has a conventional spray head connected to the container so that with actuation of the trigger a mixture of ozonated water and gas is sprayed from the nozzle.

The dispenser comprises two one-way valves on its lower surface, one for the outflow of water to the base station, and another for the inflow of ozonated water from the base station. Each of these valves co-operates with a respective valve located on the base station. As well as adding to the cost and complexity of the device, wear and/or failure of one of the valves located on the bottom surface of the dispenser could lead to leakage of ozonated water from the dispenser. Furthermore, the rate of decay of ozone within the water stored in the dispenser is fairly rapid, and so the dispenser needs to be returned to the base station after around 15 minutes to replenish the amount of ozone within the stored water.

Other spray dispensers are know from EP 1741676, JP 2004129954, and US 2004211676.

It is an aim of the present invention to provide an improved spray dispenser.

The present invention provides a spray dispenser comprising a housing containing a reservoir for storing liquid, an electrolytic cell for receiving liquid from the reservoir and increasing the level of oxidative properties in the liquid, and means for circulating fluid between the reservoir and the electrolytic cell, and a nozzle for dispensing liquid from the reservoir.

The spray dispenser thus comprises a closed loop fluid circuit, comprising the reservoir, electrolytic cell and fluid circulating means, to reduce the risk of fluid leaking from the spray dispenser and providing a compact spray dispenser.

The electrolytic cell is preferably arranged to generate hydrogen peroxide from water received from the reservoir, which has a much slower decay rate in water than ozone. Therefore, the present invention also provides a spray dispenser comprising a housing containing a reservoir for storing water, an electrolytic cell for receiving water from the reservoir and an oxygen-containing gas, and generating hydrogen peroxide therefrom, and means for circulating fluid between the reservoir and the electrolytic cell to increase the concentration of hydrogen peroxide within the stored water, and a nozzle for dispensing hydrogen peroxide-bearing water from the reservoir.

The electrolytic cell preferably comprises a gas diffusion cathode, a membrane and an anode, with the spray dispenser comprising means for supplying an oxygen-containing gas to one side of the cathode, and the means for circulating fluid configured to convey water between the other side of the cathode and the membrane. The membrane preferably comprises a proton exchange membrane.

The means for circulating water is preferably configured to convey water from the reservoir to the cathode at a first flow rate to generate hydrogen peroxide at the cathode, and to the anode at a second flow rate greater than zero and different from the first flow rate. Thus the present invention also provides a spray dispenser comprising a water reservoir, an electrolytic cell comprising a cathode, an anode and a membrane located between the cathode and the anode and defining a cathode chamber and an anode chamber, means for supplying an oxygen-containing gas to the cathode, and means for conveying water from the reservoir to the cathode chamber at a first flow rate to generate hydrogen peroxide at the cathode, and to the anode chamber at a second flow rate greater than zero and different from the first flow rate. The water conveying means preferably comprises a structure having an inlet connected to the reservoir, a first outlet connected to the cathode chamber and a second outlet connected to the anode chamber. These outlets preferably have different sizes. The structure preferably comprises a manifold, which preferably forms part of the electrolytic cell.

The means for supplying an oxygen-containing gas preferably comprises a fan for blowing air over said one side of the cathode. The means for circulating fluid is preferably configured to convey water between the anode and the membrane.

The housing preferably defines a base and a body extending upwardly from the base, the electrolytic cell being located in the base.

The spray dispenser preferably comprises a water softener for receiving water from the reservoir and outputting softened water to the electrolytic cell. The water softener preferably comprises ion-exchange material for removing transition metal ions from the water received from the reservoir. Thus, the spray dispenser preferably comprises ion-exchange material for receiving water from the reservoir, removing transition metal ions from the received water, and outputting water depleted in transition metal ions to the electrolytic cell. The softener is preferably in the form of a removable cartridge, and preferably forms at least part of the bottom surface of the base. The water softener may be shaped to define an aperture for receiving an electrical connector for supplying power to the dispenser. The spray dispenser preferably comprises a manually operable catch for releasably retaining the water softener on the dispenser.

The spray dispenser preferably comprises a spout through which water is introduced into the reservoir, the spout being moveable relative to the reservoir between an open position and a closed position. The reservoir preferably comprises means for isolating the fluid outlet from the fluid inlet when the spout is in the closed position. Thus, the present invention also provides a spray dispenser comprising a reservoir for storing a liquid, a nozzle for dispensing liquid from the reservoir, and a spout through which the reservoir is replenished with liquid, the spout comprising a fluid inlet and a fluid outlet, the spout being moveable relative to the reservoir between an open position and a closed position, the reservoir comprising means for isolating the fluid outlet from the fluid inlet when the spout is in the closed position.

The spout preferably comprises a funnel-shaped wall having a relatively wide part comprising the fluid inlet and a relatively narrow part comprising the fluid outlet. The fluid outlet preferably extends partially about the relatively narrow part of the wall. The means for isolating the fluid outlet from the fluid inlet preferably comprises a spindle arranged to receive the relatively narrow part of the wall. The spindle may comprise an annular sealing member extending thereabout for engaging the wall of the spout. The spindle may comprise a conical surface extending towards the fluid inlet for directing fluid towards the fluid outlet. The spout is preferably rotatably connected to the reservoir. The spout and the reservoir preferably together define an air bleed which is closed when the spout is in the closed position. The spout preferably extends rearwardly from the reservoir, and is preferably located opposite the nozzle. The spout is preferably substantially orthogonal to the reservoir. The spout is preferably connected to the inner surface of the reservoir. The spout may comprise a lug projecting radially therefrom, with the reservoir comprising a helical groove on the inner surface thereof for receiving the lug. The helical groove preferably extends about 90° around the inner surface of the reservoir.

The spout preferably comprises a gas passageway through which air enters the reservoir as liquid is sprayed from the nozzle. The gas passageway preferably comprises a valve for permitting air to enter the reservoir during the spraying of liquid therefrom, and for permitting a gaseous by-product of the oxidant generation by the electrolytic cell to be emitted from the reservoir. Thus the present invention also provides a spray dispenser comprising a reservoir for storing a liquid, means for receiving liquid from the reservoir and generating an oxidant therefrom, means for returning oxidant-bearing liquid to the reservoir, a nozzle for dispensing oxidant-bearing liquid from the reservoir, and a valve for permitting air to enter the reservoir during the spraying of oxidant-bearing liquid therefrom, and for permitting a gaseous by-product of the oxidant generation to be emitted from the reservoir.

The valve preferably comprises a body formed from flexible material defining a slot which is openable to permit air to enter the reservoir during the spraying of oxidant-bearing liquid. This body may have a duck bill configuration. The valve is preferably formed from elastomeric material. The valve is preferably located in a gas passageway comprising a valve seat, the valve being moveable away from the valve seat to permit the gaseous by-product to be emitted from the reservoir. The valve preferably comprises an annular surface for engaging the valve seat.

The housing preferably contains control circuitry for operating the electrolytic cell. The control circuitry is preferably configured to control the duration of the operation of the electrolytic cell depending on the time elapsed since the movement of the spout. A sensor may be provided for detecting movement of the spout, and for outputting a signal indicative thereof to the control circuitry. Alternatively, or additionally, the control circuitry may be configured to control the operation of the electrolytic cell depending on the amount of liquid that has been dispensed since the movement of the spout.

The means for circulating fluid preferably comprises a motorised pump.

The spray dispenser is preferably in the form of a hand-held spray dispenser.

The present invention also provides a handheld spray dispenser comprising a body; a head having a front portion comprising a nozzle and a motorised pump, and a rear portion having a lower surface which supports the dispenser on a user's hand during use; the body comprising a reservoir and a trigger located beneath the front portion of the head and which is actuable by the index finger of the user's hand to operate the pump to convey liquid from the reservoir to the nozzle, the trigger being shaped to provide a ledge for supporting the index finger of the user.

The ledge is preferably substantially orthogonal to the body of the dispenser, and preferably extends about the base of the trigger. The ledge is preferably substantially C-shaped. The body preferably comprises a concave portion located beneath the trigger for accommodating another finger of the user's hand. The vertical distance between the ledge and the lower surface of the rear portion of the head is preferably in the range from 20 to 30 mm. The spray dispenser preferably comprises control circuitry for operating the pump in response to depression of the trigger towards the body. The control circuitry is preferably arranged to delay operation of the pump for a period of time in the range from 0.5 to 2 seconds following depression of the trigger, to continue operation of the pump for a period of time in the range from 0.5 to 2 seconds following release of the trigger, and/or to operate the pump in response to a depression of the trigger towards the body in the range from 2 to 5 mm.

The present invention also provides apparatus comprising a spray dispenser as aforementioned and a base station for receiving the spray dispenser and for supplying electrical power to the electrolytic cell. The base station is preferably configured to support the cell during the supply of electrical power thereto so that the cell is inclined to the horizontal, preferably at an angle in the range from 6 to 20° to the horizontal. The base station preferably comprises an inclined support surface for supporting the cell during the supply of electrical power thereto. The spray dispenser preferably comprises a battery and a battery charger arranged to receive electrical power from the base station.

The present invention thus also provides apparatus for generating hydrogen peroxide, comprising a water reservoir; an electrolytic cell in the form of a laminated body comprising a cathode, an anode and a membrane sandwiched between the cathode and the anode and defining a cathode chamber and an anode chamber; means for supplying water from the reservoir to the cathode chamber and the anode chamber; means for supplying an oxygen-containing gas to the cathode to generate hydrogen peroxide at the cathode; and a support device for supplying electrical power to the cell during the generation of hydrogen peroxide and configured to support the cell during the supply of electrical power thereto so that the cell is inclined to the horizontal.

An embodiment of the invention will now be described with reference to the accompanying drawings in which:
Figure 1 is a side view of a spray dispenser located on a base station;
Figure 2 is a top view of the spray dispenser of Figure 1;
Figure 3 is a section through the spray dispenser and base station of Figure 1;
Figure 4(a) is a perspective view of the rear section of the casing of the spray dispenser of Figure 1, showing the spout in a closed position;
Figure 4(b) is a perspective view similar to Figure 4(a), showing the spout in an open position;
Figure 5(a) is a section through the rear section of the casing of the spray dispenser of Figure 1, showing the spout in the closed position;
Figure 5(b) is a sectional view similar to Figure 5(a), showing the spout in an open position;
Figure 6 is a schematic of a fluid recirculation system within the spray dispenser of Figure 1;
Figure 7 is a sectional view of the electrochemical cell of the spray dispenser of Figure 1; and
Figure 8 is an exploded view of the base station of Figure 1.

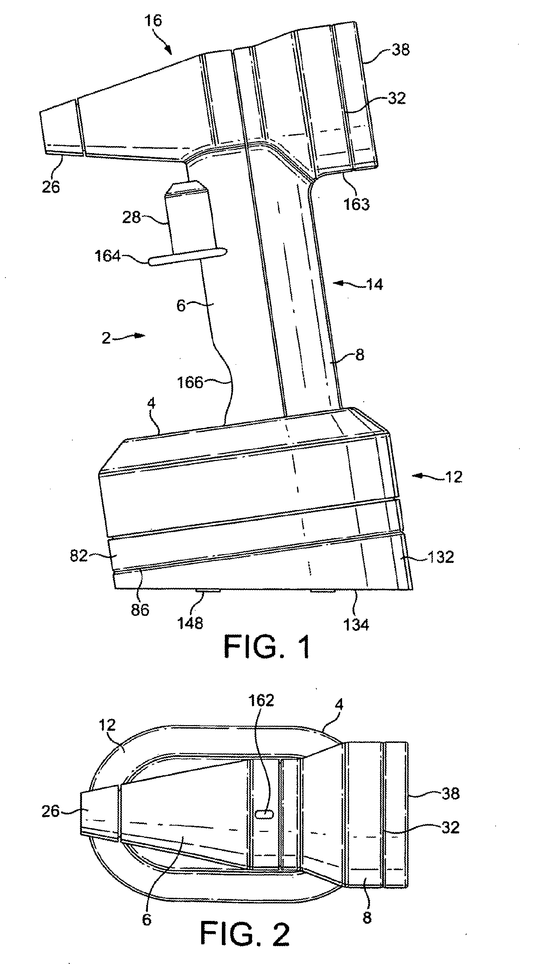

A spray dispenser according to the invention is shown in Figures 1 and 2. The dispenser 2 is arranged to dispense a sterilising liquid for sterilising a surface. In this embodiment, the dispenser 2 is arranged to generate hydrogen peroxide from water introduced to the dispenser 2 and to spray hydrogen peroxide-bearing water upon actuation by a user.

The dispenser 2 comprises a housing 4 having a front section 6 and a rear section 8 connected to and contiguous with the front section 6 so that the outer surface of the front section 6 is flush with the outer surface of the rear section 8. Preferably, the front section 6 and the rear section 8 of the housing 4 are both formed from plastics material, with the rear section 8 of the housing 4 formed preferably from transparent plastics material. The housing 4 is shaped to define a base 12, a body 14 extending upwardly from the base 12, and a head 16. The front section 6 is shaped to define the base 12, whereas the front section 6 and the rear section 8 of the housing 4 together define the body 14 and the head 16. The body 14 is preferably substantially orthogonal to the base 12. The head 16 comprises a nozzle 26 from which a liquid is dispensed in the form of a spray. A trigger 28 for actuating the spraying of liquid from the nozzle 26 is located on the body 14, directly beneath the head 16.

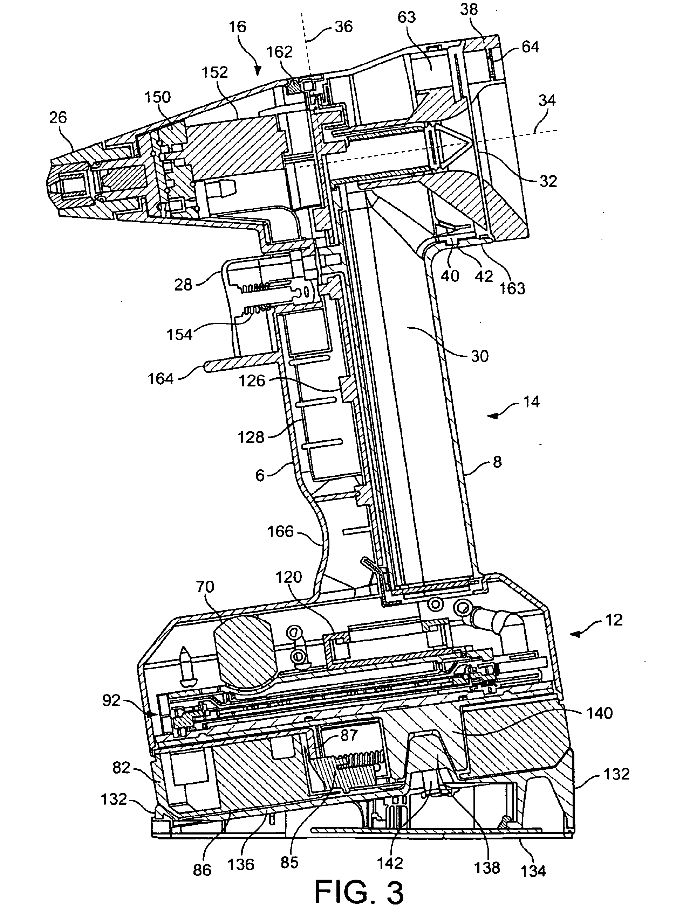

Figure 3 illustrates the interior components of the spray dispenser 2 of Figures 1 and 2. The rear section 8 of the housing 4 defines a reservoir 30 for storing the liquid to be dispensed from the nozzle 26, which in this example is an aqueous solution. The reservoir 30 preferably has a capacity in the range from 100 to 200 ml, and in this example the reservoir 30 has a capacity of around 150 ml. The rear section 8 of the housing 4 defines a substantially circular aperture 32 through which liquid, in this example water, is introduced to the reservoir 30. The aperture 32 preferably lies opposite the nozzle 26, and in a plane having a normal axis 34 which is substantially orthogonal to the longitudinal axis 36 of the body 14. A spout 38 is located within the aperture 32. The spout 38 is connected to the rear section 8 of the housing 4 so that it is moveable relative to the reservoir 30 between a closed position in which the introduction of water into the reservoir 30 from the spout 38 is inhibited, and an open position in which water may enter the reservoir 30 from the spout 38. In this example, the spout 38 is internally connected to the reservoir 30. The spout 38 has a radially protruding lug 40 that is located within a closed, helical groove 42 formed on the inner surface of the rear section 8 of the housing 4 so that with rotation of the spout 38 relative to the housing 4 the spout 38 moves along the axis 34. The helical groove 42 preferably extends around 90° around the inner surface of the reservoir 30.

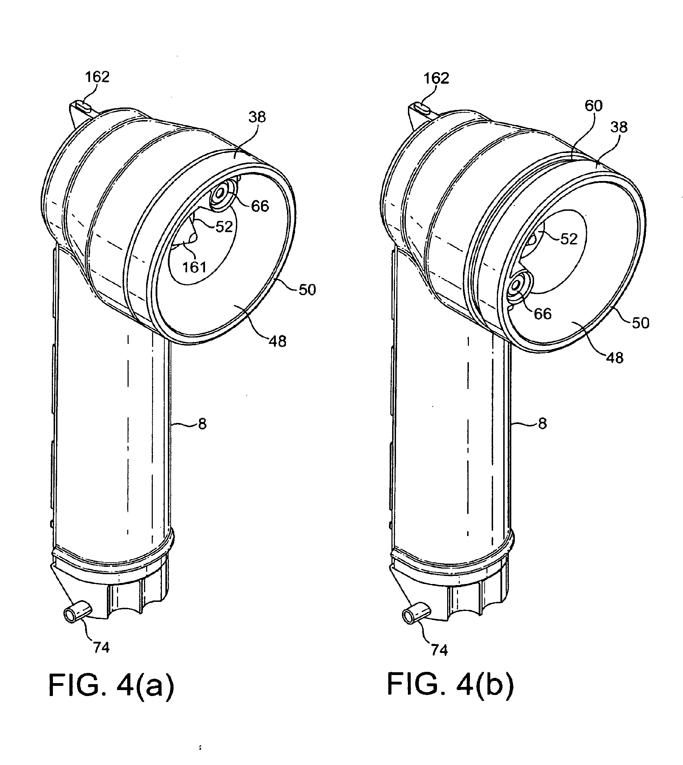

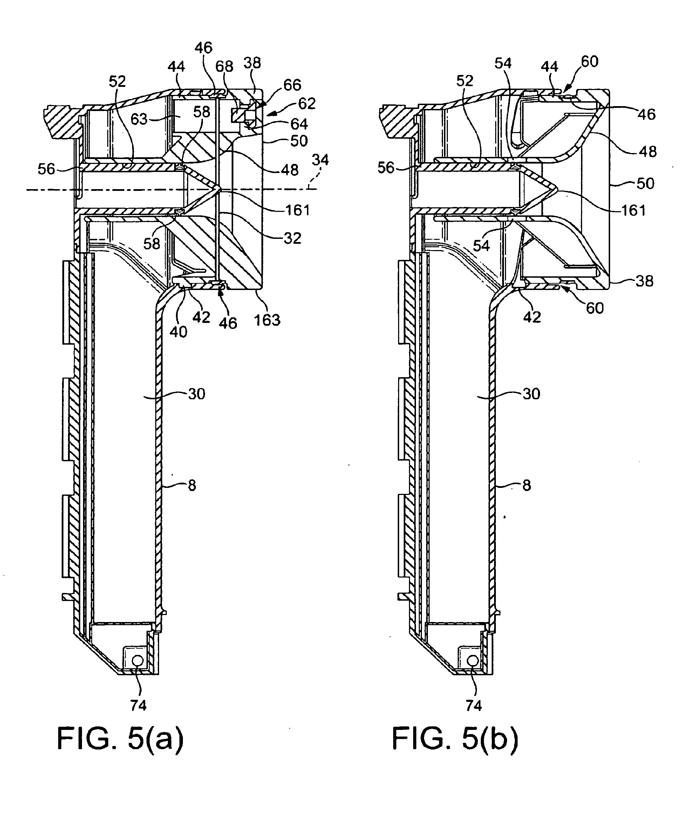

Figures 4(a) and 5(a) illustrate in more detail the rear section 8 of the housing 4. The spout 38 comprises a substantially cylindrical outer wall 44 which carries the radially protruding lug 40. The outer wall 44 is preferably substantially flush with the outer surface of the rear section 8 of the housing 4. An annular sealing member 46, preferably in the form of an elastomeric O-ring, extends about the outer wall 44 to form a substantially air-tight seal with the inner surface of the rear section 8 of the housing 4 when the spout 38 is in the closed position illustrated in Figures 4(a) and 5(a).

The outer wall 44 of the spout 38 is connected to, and extends about, a funnel-shaped inner wall 48 of the spout 38. The inner wall 48 has a relatively wide part connected to the outer wall 44 and providing a fluid inlet 50 of the spout 38, and a relatively narrow part 52 extending into the reservoir 30. As illustrated in Figure 5(b), the relatively narrow part 52 of the inner wall 48 comprises at least one fluid outlet 54 extending partially thereabout. A plurality of fluid outlets 54 may be provided in a co-planar, angularly spaced arrangement about the relatively narrow part 52 of the inner wall 48. In this example, the spout 38 comprises three fluid outlets 54.

A spindle 56 is located within the relatively narrow part 52 of the inner wall 48. The spindle 56 extends from the rear section 8 of the housing 4 towards the aperture 32. An annular sealing member 58, also preferably in the form of an elastomeric O-ring, extends about the spindle 56 to engage and form a substantially air-tight seal with the inner surface of the relatively narrow part 52 of the inner wall 48 when the spout 38 is in the closed position. In this closed position, the fluid outlets 54 are located to the left (as illustrated) of the sealing member 58 so that the fluid outlets 54 are isolated from the fluid inlet 50 by the sealing member 58.

As mentioned above, the helical groove 42 of the connection between the rear section 8 of the housing 4 and the spout 38 is shaped so that with rotation of the spout 38, the spout 38 is translated along axis 34. Figures 4(b) and 5(b) illustrate the spout 38 in an open position following a 90° anticlockwise (as illustrated) rotation of the spout 38. In this open position, the fluid outlets 54 are at least partially located to the right (as illustrated) of the sealing member 58 so that the fluid outlets 54 are in fluid communication with the fluid inlet 50. In addition, the sealing member 46 extending about the outer wall 44 of the spout 38 is now spaced from the inner wall of the rear section 8 of the housing 4 so that an air bleed 60 is formed between the spout 38 and the rear section 8 of the housing 4.

As illustrated in Figures 4(a) and 5(a), the dispenser 2 includes a valve assembly 62. The function of the valve assembly 62 is to allow gas both to escape from the reservoir 30 and to enter the reservoir 30 when the spout 38 is in the closed position, thereby equalising the gas pressure between the reservoir 30 and the external environment. In this example, the valve assembly 62 is located within a gas passageway 63 passing through the spout 38 between the outer wall 44 and the inner wall 48 of the spout 38.

The gas passageway 63 is preferably located in the upper portion of the spout 38 when the spout 38 is in the closed position. The spout 38 is shaped to define a valve seat 64 which extends about the gas passageway 63, and which receives a valve body 66. The valve body 66 is formed from elastomeric material, and is arranged to move relative to the valve seat 64 depending on a pressure differential across the valve body 66. In this example, the valve body 66 comprises a first portion having a duck bill configuration, comprising a pair of semi-circular valve lips 68 defining a slot opening which is normally closed by the lips 68, and second portion having an umbrella-type configuration for engaging the valve seat 64. When the gas pressure within the reservoir 30 is lower than atmospheric pressure, the force exerted on the valve body 66 by the external atmosphere causes the first portion of the valve body 66 to deform to open the slot opening and permit gas to enter the reservoir 30 from the external environment. When the gas pressure within the reservoir 30 is greater than atmospheric pressure, the force exerted on the valve body by the gas within the reservoir 30 causes the second portion of the valve body 66 to move relative to the valve seat 64 to permit gas to pass through the gas passageway 63 to the atmosphere.

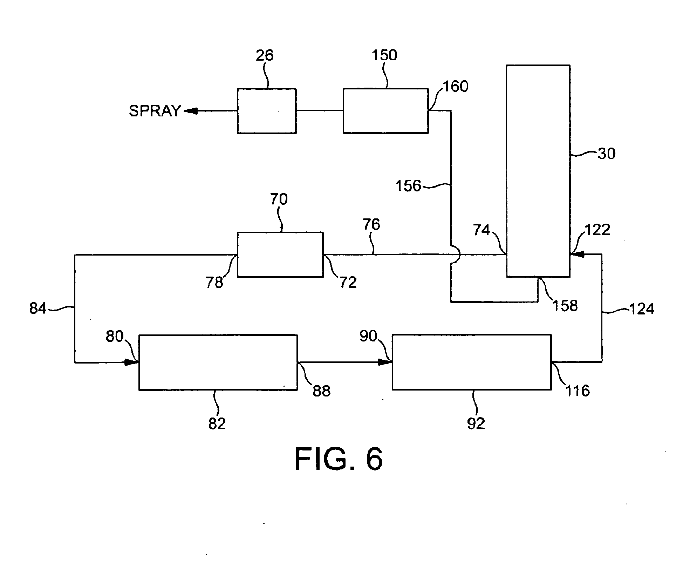

The reservoir 30 forms part of a fluid recirculation system located within the housing 4 of the dispenser 2. This recirculation system is illustrated schematically in Figure 6. In addition to the reservoir 30, the recirculation system comprises a fluid pump 70 for pumping fluid through the recirculation system. As illustrated in Figure 3, the fluid pump 70 is preferably located within the base 12 of the dispenser 2. The fluid pump 70 is arranged to pump fluid through the recirculation system at a rate in the range from 10 to 100 ml/min and at a pressure sufficient to force water through the recirculation system. In this example, the fluid flows through the recirculation system at a rate of around 32 ml/min. The fluid pump 70 is preferably a motorised pump, for example a positive displacement pump. The fluid pump 70 has an inlet 72 connected to a first fluid outlet port 74 of the reservoir 30 by a conduit 76. The first fluid outlet port 74 is preferably located towards the bottom of the rear section 8 of the housing 4, as illustrated in Figures 4(a) and 5(a), so that the fluid can be circulated within the fluid circulation system when the reservoir 30 contains only a relatively small amount of liquid.

The outlet 78 of the fluid pump 70 is connected to the inlet 80 of a water softener 82 by conduit 84. The water softener 82 is in the form of a cartridge removably connected to the base 12 of the dispenser 2. A spring-loaded catch mechanism 85 connected to the base 12 engages a downwardly extending rib 87 of the water softener 82 to hold the water softener 82 against the base 12. As illustrated in Figure 3, the water softener 82 is removably connected to the base 12 so that the water softener 82 provides the bottom surface 86 of the dispenser 2, which facilitates the connection of the water softener 82 to the dispenser 2 and its subsequent replacement. As is common practice, the water softener 82 contains a bed of ion-exchange resin beads which attract calcium and magnesium ions within the circulating water and replace them with sodium ions. A second bed of ion-exchange material may be provided for removing transition metal ions from the circulating water. This second bed may be located within a separate compartment of the water softener, or it may be mixed with the ion-exchange resin beads for attracting calcium and magnesium. As another alternative, this second bed may be housed within a separate cartridge removably connected to the dispenser 2.

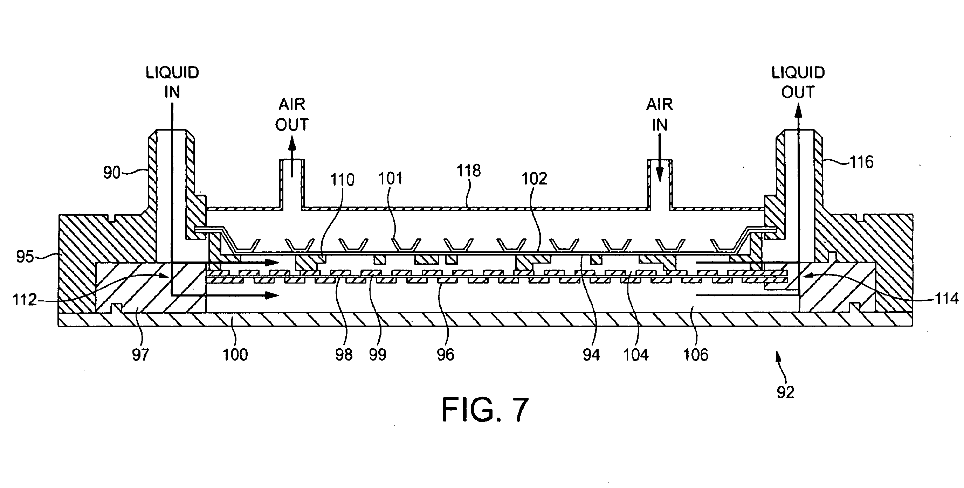

The outlet 88 of the water softener 82 is connected to the inlet 90 of an electrolytic cell 92 located within the base 12 of the dispenser 2. A sectional view of the cell is shown in Figure 7. The cell 92 is in the form of a laminated body arranged within the base 12 so that it lies in a plane which is substantially parallel to the bottom surface 86 of the base 12. The cell 92 comprises a gas permeable cathode 94, an anode 96 and a membrane 98 located between the cathode 94 and the anode 96. The cathode 94 is preferably provided by a rectangular carbon cloth impregnated or coated with carbon particles. The perimeter of the cathode 94 is held by a first plastics moulding 95. The anode 96 is preferably formed from a rectangular titanium mesh coated with a conductive metal oxide, for example tantalum oxide, iridium oxide and/or ruthenium oxide, or other corrosion-resistant conductive material. The perimeter of the anode is held by a second plastics moulding 97 located beneath the first plastics moulding 95 and which also holds the perimeter of the membrane 98 so that the upper (as illustrated) surface of the anode 96 is in close contact with the lower surface of the membrane 98. A perforated plastics membrane support 99 may be optionally provided in close contact with the upper (as illustrated) surface of the membrane 98. A base 100 is connected to the bottom of the second moulding 97 to close the bottom of the cell 92.

The cathode 94 and the anode 96 are connected to a DC power source (not shown in figure 7) for applying an appropriate current and voltage across the electrodes during use of the cell 92. The cathode 94 is connected to the power source by a rectangular metallic plate 101, which in this example is formed from brass, juxtaposed with the cathode 94 and comprising an array of apertures. As explained in more detail below, a microporous fluorocarbon layer 102 is sandwiched between the cathode 94 and the metallic plate 101, and substantially covers the upper (as illustrated) surface of the cathode 94. The perimeters of the metallic plate 101 and the layer 102 also may be held by the first moulding 95. Leads (not shown) connect the anode 96 and the metallic plate 101 to the power source.

In this example, the membrane 98 is a proton conducting membrane, which is preferably formed from a Nafion™ film. The membrane 98 defines within the cell 92 a cathode chamber 104 and an anode chamber 106. The cathode chamber 104 is located between the lower (as illustrated) surface of the cathode 94 and the upper surface of the membrane 98, and the anode chamber 106 is located between lower surface of the membrane 98 and the upper surface of the base 100 of the cell 92. An apertured plastics spacer 110 is positioned between the cathode 94 and the membrane 98 (or optional membrane support 99) to set the distance between the cathode 94 and the membrane 98.

The mouldings 95, 97 are shaped to define an inlet manifold - indicated at 112 in Figure 7 - which has an inlet arranged to receive water from the inlet 90 of the cell 92, and a plurality of outlets arranged to convey water into a respective one of the cathode chamber 104 and the anode chamber 106. The mouldings 95, 97 are also shaped to define an outlet manifold - indicated at 114 in Figure 7 - having a plurality of inlets each arranged to receive water from a respective one of the cathode chamber 104 and the anode chamber 106 and an outlet arranged to convey water to the outlet 116 from the cell 92. Thus, the softened water received from the water softener 82 is supplied both to the cathode chamber 104 as a catholyte and to the anode chamber 106 as an anolyte. The cell 92 is configured to supply catholyte to the cathode chamber 104 at a first flow rate, preferably greater than 20 ml/min and in this example around 30 ml/min, and to supply anolyte to the anode chamber 106 at a second, non-zero flow rate lower than the first flow rate, preferably less than 5 ml/min and in this example around 2 ml/min. The variation in the flow rate of water through the cathode chamber 104 and the anode chamber 106 may be generated in one of a number of different ways. In this example, the outlets of the inlet manifold 112 have respective different sizes. Alternatively, a flow restrictor may be located between the inlet manifold 112 and the anode chamber 106 to restrict the flow of water through the anode chamber 106. The spacer 110 is shaped to create an even flow of water across the lower (as illustrated) surface of the cathode 94 whilst inhibiting contact between the cathode 94 and the membrane 98.

As an alternative to supplying the softened water received from the water softener 82 to the cathode chamber 104 in parallel with its supply to the anode chamber 106, the softened water may be conveyed through these chambers in series. For example, the softened water may be conveyed through the cathode chamber 104 and then through the anode chamber 106, or vice versa. Whilst this arrangement decreases the performance of the cell 92, the structure of the cell 92 is somewhat simplified.

The cell 92 further comprises an air chamber 118 for supplying an oxygen-containing gas, in this example air, to the cathode 94. As illustrated in Figure 3, a fan 120 is mounted on the cell 92 for generating a flow of air through the air chamber 118. The fan 120 is configured to generate an air flow through the air chamber 118 having a flow rate of around 3 l/min, and a pressure in the range of 1 to 3 mbar. A pressure relief valve may be provided to allow excess air to be discharged from the air chamber 118.

Returning to Figure 6, the fluid discharged from the outlet 116 of the cell 92 is conveyed to a fluid inlet port 122 of the reservoir 30 by conduit 124. The fluid inlet port 122 is preferably located opposite the first fluid outlet port 74 on the rear section 8 of the housing 4.

The operation of the fluid pump 70, the cell 92 and the fan 120 is controlled by electronic control circuitry 126 located within the front section 6 of the housing 4, as illustrated in Figure 3. The control circuitry 126 comprises a microprocessor and is mounted on a printed circuit board, which in turn may be mounted on the front of the rear section 8 of the housing 4 so as to be located wholly within the front section 6 of the housing 4.

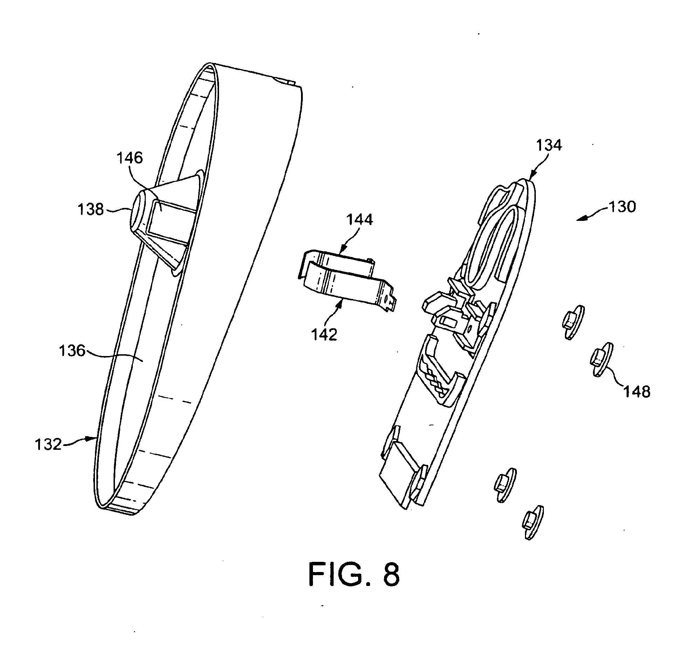

A battery pack 128 is connected to the printed circuit board. The battery pack 128 is preferably rechargeable, and preferably comprises a single lithium-ion cell which produces an output having a voltage of 2.4-3.6 V when fully charged. The control circuitry 126 preferably comprises a charge circuit for charging the battery pack. The control circuitry 126 receives electrical power for charging the battery pack 128 from a base station 130 configured to receive the base 12 of the dispenser 2. With reference to Figure 3 and Figure 8, the base station 130 comprises a body having a substantially semi-circular side wall 132 connected to and upstanding from a base 134. An inclined surface 136 is integral with and surrounded by the side wall 132, and defines with the side wall 132 a cavity for receiving the bottom surface 86 of the base 12 of the dispenser 2 and retaining the dispenser 2 on the base station 130. The inclined surface 136 is preferably inclined at an angle in the range from 6 to 20° to the horizontal, and in this example is inclined at an angle of around 8° to the horizontal. A domed connector 138 extends upwardly from the inclined surface 136 to mate with a socket 140 located on the base 12 of the dispenser 2 and which passes through an aperture formed in the water softener 82. A pair of metallic connectors 142, 144 is upstanding from the base 134, each connector 142, 144 passing through a respective aperture 146 in the domed connector 138 to contact electrical connectors (not shown) located in the socket 140 and arranged to supply power to the control circuit 126. The control circuit 126 comprises a sensor (not shown) which outputs a signal when the dispenser 2 is positioned correctly on the base station 130, that is, with the connectors 142, 144 contacting the electrical contacts on the base 12. Feet 148 may be connected to the bottom surface of the base 134 of the base station 130 for engaging a work counter or other surface on which the base station 130 is situated. A mains cable (not shown) extends from the base station 130 and terminates in a plug for connecting the base station 130 to a mains socket.

Returning to Figure 3 and Figure 6, the dispenser 2 comprises a second fluid pump 150 for supplying water from the reservoir 30 to the nozzle 26. The second fluid pump 150 is located in the front section 6 of the casing 4, preferably immediately behind the nozzle 26. The second fluid pump 150 is preferably a gear pump, or another positive displacement pump, operated by a motor 152 which is also located in the front section 6 of the casing 4. The operation of the motor 152 is controlled by the control circuitry 126 in response to actuation of the trigger 28. The trigger 28 is biased away from the body 14 of the dispenser 2 towards an "off' position by a spring 154 or other resilient element, in which position the second fluid pump 150 is inactive. A flexible conduit 156 for supplying water to the second fluid pump 150 is connected between a second fluid outlet port 158 of the reservoir 30 and the inlet 160 of the second fluid pump 150. The second fluid outlet port 158 is preferably located towards the bottom of the rear section 8 of the casing 4, and may be located alongside of beneath the first fluid outlet port 74.

The operation of the dispenser 2 will now be described in detail. To fill the reservoir 30, the user removes the dispenser 2 from the base station 130, and holds the body 14 of the dispenser 2 in one hand so that the axis 34 of the spout 38 is roughly vertical. With the thumb and forefinger of the other hand, the user rotates the spout 38 anticlockwise by around 90° to move the spout 38 from the closed position shown in Figure 4(a) and Figure 5(a) to the open position shown in Figure 4(b) and Figure 5(b). The user then pours water from a tap, dispenser or other source of water into the spout 38. The water is conveyed by the inner wall 48 of the spout 38 from the fluid inlet 50 of the spout 38 to the fluid outlets 54, through which the water enters the reservoir 30. As illustrated in Figures 5(a) and 5(b), the end 161 of the spindle 56 is conical in shape so as to guide the water into the fluid outlets 54. As the air bleed 60 is open when the spout 38 is in an open position, air is displaced from the reservoir 30 through the air bleed 60 as the reservoir 30 becomes filled with water.

The user is instructed to fill the reservoir 30 each time the spout 38 is moved to the open position. When the reservoir 30 has been filled, the user rotates the spout 38 clockwise to return the spout 38 to the closed position and close the air bleed 60. For reasons discussed in more detail below, the control circuitry 126 preferably comprises a sensor for detecting the movement of the spout 38 from the closed position to the open position. This sensor may be conveniently located on the rear surface of the printed circuit board.

The user then places the dispenser 2 on the base station 130, to which electrical power is being supplied from the mains socket. The control circuit 126 comprises a sensor (not shown) which outputs a signal when the dispenser 2 is positioned correctly on the base station 130, that is, with the connectors 142, 144 contacting the electrical contacts on the base 12. Upon receipt of this signal, the control circuit 126 uses the electrical power received from the base station 130 to operate the fluid pump 70 and the fan 120, and to activate the cell 92. Depending on the voltage of the battery pack 128, the electrical circuit may also control the charge circuit to recharge the battery pack 128. Another sensor may be provided for outputting a signal indicative of the current voltage of the battery pack 128, which is used by the control circuit 126 to determine whether the battery pack 128 requires recharging.

The operation of the fluid pump 70 causes water to be circulated through the fluid recirculation system. Water flows from the first fluid outlet port 74 of the reservoir 30 to the inlet 72 of the fluid pump 70, and then from the outlet 78 of the fluid pump 70 to the inlet 80 of the water softener 82 at a rate of around 32 ml/min. Within the water softener 82, the water is conveyed through the bed of ion-exchange resin beads so that calcium and magnesium ions within the water are replaced with sodium ions.

Having passed through the water softener 82, the softened water enters the electrolytic cell 92 through the inlet 90 thereof. The softened water enters the inlet manifold 112 defined by the first and second plastics mouldings 95, 97 and which divides the softened water into a first stream and a second stream. Due to the different sizes of the outlets of the inlet manifold 112, the first stream is conveyed into the cathode chamber 104 as a catholyte at a rate of around 30 ml/min, and the second stream is conveyed into the anode chamber 106 as an anolyte at a rate of around 2 ml/min.

The operation of the fan 120 by the control circuitry 126 generates a flow of air through the air chamber 118. Oxygen molecules within the air chamber 118 pass through the apertures in the metallic plate 101 and the pores in the microporous layer 102 to enter the pores of the carbon cloth forming the cathode 94. The provision of the microporous layer 102 between the metallic plate 101 and the cathode 94 provides a physical and chemical barrier to the passage of softened water from the cathode chamber 104 to the air chamber 118. By providing such a barrier between the cathode chamber 104 and the air chamber 118, the leakage of water from the fluid recirculation system through the cathode 94 and the air chamber 118 is inhibited. Furthermore, as the air within the air chamber 118 is not required to act as a pneumatic barrier to the entry of water into the air chamber 118 from the cell 92, the pressure of the air stream flowing through the air chamber 118 can have a relatively low value, for example in the range from 1 to 3 mbar, that is sufficient to supply oxygen molecules to the cathode 94 at an acceptable rate. This enables a relatively low cost and relatively small fan 120 to be used to generate the air flow within the air chamber 118.

The control circuitry 126 activates the cell 92 by applying an electrical potential across the cathode 94 and the anode 96. The cell 92 is activated a period of time, in this example around 3 to 4 seconds, after the operation of the fluid pump 70 has commenced so that water is already flowing through the cell 92 when the cell 92 is activated. The operation of the fan 120 is commenced before, in this example around 3 to 4 seconds before, the operation of the fluid pump 70 to prevent the cathode 94 from becoming flooded with water. At the anode 96 of the activated cell 92, the softened water is oxidised to form oxygen and protons (hydrogen ions) according to the following reaction:

*2H₂O* → *O₂* + *4H*⁺ + *4e⁻*

The meshed nature of the anode 96 provides a large number of edges at which oxygen gas is released. The protons migrate across the membrane 98 towards the cathode 94, at which a three phase gas-liquid-solid interface exists between the air entering the cathode 94 from the air chamber 118 and the softened water entering the cathode 94 from the cathode chamber 104. At the interface, the oxygen is reduced to hydrogen peroxide, which reaction can be expressed simply as:

*2H⁺* + *O₂* + 2e⁻ → *H₂O₂*

The current density at the cathode 94 is controlled to inhibit the undesirable formation at the cathode 94 of hydrogen gas (H₂) from the reduction of water. First, the control circuitry 126 is arranged to apply a relatively low potential difference in the range from 10 to 20 V across the electrodes of the cell 92. Secondly, within the cathode chamber 104 the spacer 110 serves to generate a relatively even flow of water across the lower surface of the cathode 94, which in turn generates a relatively even current distribution across the cathode 94. This inhibits the formation of isolated "pockets" of relatively high current density at regions of the cathode 94 where the flow rate of water is relatively low.

During use of the cell 92, the membrane 98 is constantly in contact with water, which causes the membrane 98 to swell. The apertures formed in the spacer 110, and/or the optional membrane support 99, allow the membrane 98 to expand upwards through these apertures towards the cathode 94. The size and distribution of these apertures results in a relatively uniform, small expansion of the membrane 98 through each aperture. The spacing between the cathode 94 and the membrane 98 is selected so that the expanded membrane 98 does not come into contact with the cathode 94 and become damaged.

The replacement of calcium ions with sodium ions within the water softener 82 serves to prolong significantly the working life of the cell 92. The presence of calcium ions within the catholyte flowing through the cathode chamber 104 would otherwise result in the deposition of calcium carbonate on the cathode 94, blocking the pores of the carbon cloth and preventing oxygen from forming a gas-liquid-solid interface within the cathode 94.

The outlet manifold 114 thus receives a stream of hydrogen peroxide-bearing water from the cathode chamber 104 and a stream of oxygen-bearing water from the anode chamber 106. These two water streams are combined at the outlet manifold 114, and the combined water stream, containing hydrogen peroxide and bubbles of oxygen, is conveyed under the pumping action of the fluid pump 70 from the outlet 116 of the cell 92 to the fluid inlet port 122 of the reservoir 30. As a result, during the operation of the fluid recirculation system the concentration of hydrogen peroxide within the water stored in the reservoir 30 gradually increases. The bubbles of oxygen entrained within the water entering the reservoir 30 through the fluid inlet port 122 are discharged periodically from the reservoir 30 by the valve assembly 62 when the gas pressure within the reservoir 30 is sufficient to cause the second portion of the valve body 66 to move away from the valve seat 64.

As a consequence of the circulation of water between the reservoir 30 and the cell 92, the anolyte passing through the anode chamber 106 will include a gradually increasing amount of hydrogen peroxide. The hydrogen peroxide within the anolyte is oxidised to water according to the following reaction:

2*H₂O₂* → 2*H₂O* + *O₂* + 2*e⁻*

The flow rate of water through the anode chamber 106 (in this example around 2 l/min) is selected to be significantly lower than the flow rate of water through the cathode chamber 104 (in this example 30 l/min) to minimise the decomposition of hydrogen peroxide at the anode 96. The flow rate of water through the anode chamber 106 is maintained at a non-zero level as the mixing of hydrogen peroxide-rich water from the cathode chamber 104 with hydrogen peroxide-depleted water from the anode chamber 106 advantageously serves to reduce the pH of the water stored in the reservoir 30, which in turn enhances the stability of the hydrogen peroxide within the stored water (by reducing the rate of decay, or half life, of the hydrogen peroxide). In this example, the pH of the solution stored in the reservoir 30 upon completion of the hydrogen peroxide generation is preferably less than 9.5. Furthermore, the flow of anolyte through the anode chamber 106 serves to dislodge the bubbles of oxygen gas generated at the edges of the anode 96, enabling these bubbles to be carried away from the anode chamber 106 within the flow of anolyte and allowing fresh bubbles of oxygen gas to be generated at the anode 96.

The removal of oxygen bubbles from the anode chamber 106 is further assisted by the inclination of the cell 92 relative to the horizontal during the generation of hydrogen peroxide. In view of this, the control circuitry 126 is arranged to operate the fluid pump 70 and the fan 120, and activate the cell 92, only when the dispenser 2 is positioned correctly on the base station 130, that is, with the bottom surface 86 of the base 12 of the dispenser 2 fully located on and parallel to the inclined surface 136 of the base station 130. When the dispenser 2 is removed from the base station 130, the control circuitry 126 is arranged to, in turn, deactivate the cell 92 to terminate the generation of hydrogen peroxide, stop the operation of the fluid pump 70, and stop the operation of the fan 120.

The removal of transition metal ions from the circulating water also assists in minimising the rate at which hydrogen peroxide is oxidised to water. It has been found that such metal ions can act as a catalyst for the decomposition of hydrogen peroxide, and so the presence of transition metal ions within the circulating water would serve to promote undesirably the oxidation of hydrogen peroxide within the anode chamber 106.

The control circuit 126 is preferably arranged to activate the cell 92 for a period of time sufficient to generate a concentration of hydrogen peroxide within a substantially full reservoir 30, or around 150 ml of water, in the range from 0.6 to 0.65%, in this example around 0.62%. In this example, the concentration of hydrogen peroxide reaches this value in around 2 to 10 hours, preferably around 4 to 6 hours. Returning to Figure 2 and Figure 4(a), a light pipe 162 is mounted on the rear section 8 of the casing 4 so as to protrude through an aperture formed in the front section 6 of the casing 4. The light pipe 162 is connected to a green light emitting diode (LED) and a red LED of the control circuitry 126. The control circuitry 126 operates the LEDs to generate a number of visual alerts to the user of the dispenser 2. These visual alerts may include:
- a constant red light during the generation of hydrogen peroxide by the cell 92;
- a flashing red light in the event of a fault during the generation of hydrogen peroxide by the cell, for example if the reservoir 30 is empty or if exhaustion of the water softener 82 has resulted in the deposition of calcium carbonate within the cell 92;
- a flashing amber light in the event that the dispenser 2 is removed from the base station before the concentration of hydrogen peroxide within the stored water has reached a predetermined value, in the event that the concentration of hydrogen peroxide within the stored water is below, or has fallen below, that value, or in the event that the battery pack 128 requires recharging;
- a flashing green light to indicate that the spout 38 is in the open position; and
- a constant green light when the generation of hydrogen peroxide by the cell has been completed.

In this example, this predetermined value is in the range from 0.45 to 0.6%, preferably around 0.5%.

The transition from a constant red light to a constant green light alerts the user that the dispenser 2 is ready for use. To remove the dispenser 2 from the base station 130, the user grasps the body 14 of the dispenser 2, and lifts it from the base station 130. When removed from the base station 130, power is supplied to the control circuitry 126 from the battery pack 128, and is used by the control circuitry 126 to operate, inter alia, the motor 152 and the LEDs.

The dispenser 2 is designed to be held in an ergonomic and comfortable manner by the user. The dispenser 2 is designed to be held so that the rearwardly extending bottom surface 163 of the head 16 of the dispenser 2 is supported on the user's thumb, and so that the user's index finger is supported on a ledge 164 extending about the base of the trigger 28. As illustrated in Figure 1 and Figure 3, the ledge 164 is substantially orthogonal to the body 14 of the dispenser 2. The ledge 164 is substantially C-shaped, or semi-annular, so as to provide support to a range of different hand sizes. The ledge 164 is located beneath the bottom surface 163 of the head 16 so that the index finger of the user does not need to be stretched upwardly or downwardly in order to rest on the upper surface of the ledge 164. In this example, the vertical distance between the bottom surface 163 of head 16 and the upper surface of the ledge 164 of the trigger 28 is in the range from 20 to 30 mm, preferably around 26 mm. The distance between the front of the ledge 164 and the rear surface of the rear section 8 of the casing 4 is preferably in the range from 40 to 50 mm, more preferably 46 mm, so that the index finger of the user does not need to be stretched outwardly in order to rest on the upper surface of the ledge 164. The vertical distance between the ledge 164 and the upper surface of the base 12 of the dispenser is in the range from 60 to 80 mm, preferably around 67 mm, so that the other fingers of the user's hand which grip the body 14 of the dispenser 2 may be located comfortably between the ledge 164 and the base 12. The body 14 preferably includes a concave portion 166 beneath the trigger 28 for accommodating the little finger of the user's hand so that it is not required to stretch around the body 14 of the dispenser 2 to grip the dispenser 2. The maximum weight of the dispenser 2 is less than 500 g to enable the dispenser 2 to be carried easily by the user.

To dispense hydrogen peroxide-bearing water from the dispenser 2, the user aims the nozzle 26 at a surface to be sterilised and depresses the trigger 28 by a short distance, for example in the range from 3 to 5 mm, against the force of the spring 154. This depression of the trigger 28 is detected by the control circuitry 126. The control circuitry 126 uses the charge stored in the battery pack 128 to operate the motor 152 of the second fluid pump 150 to cause hydrogen peroxide-bearing water to be drawn from the reservoir 30 through the second fluid outlet port 158. The hydrogen peroxide-bearing water passes through the second fluid pump 150 and is dispensed from the nozzle 26 in the form of a spray at a rate of around 1 ml/second and at a pressure in the range from 1 to 2 bar. The spray preferably has a spray angle in the range from 60 to 70°. As hydrogen peroxide-bearing water is drawn out of the reservoir 30, a partial vacuum is created in the reservoir 30. As the pressure of the gas within the reservoir 30 decreases, the force exerted on the valve body 66 by the external atmosphere causes the first portion of the valve body 66 to deform to open the slot opening in the valve body 66 and permit air to enter the reservoir 30 from the external environment, returning the gas pressure within the reservoir 30 to around atmospheric pressure.

The spraying of hydrogen peroxide-bearing water continues until the first to occur of (i) release of the trigger 28 by the user, preferably for a time period greater than 0.5 seconds so that the dispenser 2 continues to spray hydrogen peroxide-bearing water even if the trigger 28 is momentarily released, (ii) emptying of the reservoir 30, and (iii) exhaustion of the battery pack 128.

The hydrogen peroxide stored within the reservoir 30 will gradually decay to form water and oxygen, and so with time the concentration of hydrogen peroxide within the solution sprayed from the dispenser 2 will gradually decrease. For example, it will take around 12 hours for the concentration of hydrogen peroxide to decrease from around 0.62% to the predetermined value mentioned above, in this example around 0.5%. The control circuitry 126 is configured to determine when the concentration of hydrogen peroxide has fallen to this value on a time basis, starting from the detection of the movement of the spout 38 to the open position. Once the control circuitry 126 has determined that the concentration of hydrogen peroxide has fallen to this value, the control circuitry 126 operates the LEDs to generate the flashing amber light to alert the user that the dispenser 2 should be returned to the base station 130. Upon return of the dispenser 2 to the base station 130, the control circuitry 126 reactivates the circulation system for a time period depending on (i) the time period which has elapsed since the spout 38 was opened, and (ii) the amount of hydrogen peroxide-bearing water that has been dispensed since the spout 38 was opened, which may be determined from the duration of the actuation of the trigger 28. In the event that the spout 38 is re-opened prior to the replacement of the dispenser 2 on the base station 130, the control circuitry 126 assumes that the reservoir 30 has been refilled with fresh water. Replacement of the dispenser 2 on the base station 130 also serves to recharge the battery pack 128.

The user may be instructed to change the water softener 82 on a regular basis, for example every 6 months. Alternatively, the control circuitry 126 may be arranged to monitor the amount of current drawn by the cell 92 during use, as fluctuation in the current drawn by the cell 92 may be indicative of the build-up of calcium deposits in the cell 92 due to exhaustion of the water softener 82. Depending on the current drawn by the cell 92, the control circuitry 126 may operate the LEDs to generate an alert, for example a constant amber alert, to advise the user to the condition of the water softener 82. Once the water softener 82 has been replaced, the calcium deposits in the cell 92 will be gradually removed by the (non-calcium-bearing) water flowing through the cell 92.

The invention is not limited to the specific embodiments described in detail above.

## Claims

1. A spray dispenser comprising:
a housing containing a reservoir for storing liquid, an electrolytic cell for receiving liquid from the reservoir and increasing the level of oxidative properties in the liquid, and means for circulating fluid between the reservoir and the electrolytic cell; and
a nozzle for dispensing liquid from the reservoir.

2. A spray dispenser as claimed in claim 1, wherein the electrolytic cell is arranged to generate hydrogen peroxide from water received from the reservoir.

3. A spray dispenser comprising
a housing containing a reservoir for storing water, an electrolytic cell for receiving water from the reservoir and an oxygen-containing gas, and generating hydrogen peroxide therefrom, and means for circulating fluid between the reservoir and the electrolytic cell to increase the concentration of hydrogen peroxide within the stored water; and
a nozzle for dispensing hydrogen peroxide-bearing water from the reservoir.

4. A spray dispenser as claimed in claim 2 or claim 3, wherein the electrolytic cell comprises a gas diffusion cathode, a membrane and an anode, the spray dispenser comprising means for supplying an oxygen-containing gas to one side of the cathode, and wherein the means for circulating fluid is configured to convey water between the other side of the cathode and the membrane.

5. A spray dispenser as claimed in claim 4, wherein the means for supplying an oxygen-containing gas comprises a fan for blowing air over said one side of the cathode.

6. A spray dispenser as claimed in claim 4 or claim 5, wherein the means for circulating fluid is configured to convey water between the anode and the membrane.

7. A spray dispenser as claimed in any one of claims 4 to 6, wherein the membrane comprises a proton exchange membrane.

8. A spray dispenser as claimed in any one of the preceding claims, wherein the housing defines a base and a body extending upwardly from the base, and wherein the electrolytic cell is located in the base.

9. A spray dispenser as claimed in one of the preceding claims, comprising a water softener for receiving water from the reservoir and outputting softened water to the electrolytic cell.

10. A spray dispenser as claimed in one of the preceding claims, wherein the water softener comprises ion-exchange material for removing transition metal ions from the water received from the reservoir.

11. A spray dispenser as claimed in one of claims 1 to 8, comprising ion-exchange material for receiving water from the reservoir, removing transition metal ions from the received water, and outputting water depleted in transition metal ions to the electrolytic cell.

12. A spray dispenser as claimed in any one of the preceding claims, comprising a spout through which water is introduced into the reservoir, the spout being moveable relative to the reservoir between an open position and a closed position.

13. A spray dispenser as claimed in any one of the preceding claims, wherein the housing contains control circuitry for operating the electrolytic cell.

14. A spray dispenser as claimed in claims 12 and 13, wherein the control circuitry is configured to control the duration of the operation of the electrolytic cell depending on the time elapsed since the movement of the spout.

15. A spray dispenser as claimed in claim 14, comprising a sensor for detecting movement of the spout, and for outputting a signal indicative thereof to the control circuitry.

16. A spray dispenser as claimed in any one of claims 14 to 15, wherein the control circuitry is configured to control the operation of the electrolytic cell depending on the amount of liquid that has been dispensed since the movement of the spout.

17. A spray dispenser as claimed in any one of the preceding claims, wherein the means for circulating fluid comprises a motorised pump.

18. A spray dispenser as claimed in any one of the preceding claims, in the form of a hand-held spray dispenser.

19. Apparatus comprising a spray dispenser as claimed in any one of the preceding claims and a base station for receiving the spray dispenser and for supplying electrical power to the electrolytic cell.

20. Apparatus as claimed in claim 19, wherein the spray dispenser comprises a battery and a battery charger arranged to receive electrical power from the base station.

## Patentansprüche

1. Sprühspender, der Folgendes umfasst:
ein Gehäuse, das einen Behälter zum Aufbewahren von Flüssigkeit, eine elektrolytische Zelle zum Aufnehmen von Flüssigkeit aus dem Behälter und Steigern des Niveaus der oxidativen Eigenschaften in der Flüssigkeit und Mittel zum Umwälzen von Flüssigkeit zwischen dem Behälter und der elektrolytischen Zelle enthält, und
eine Düse zum Spenden von Flüssigkeit aus dem Behälter.

2. Sprühspender nach Anspruch 1, wobei die elektrolytische Zelle dafür angeordnet ist, aus aus dem Behälter empfangenem Wasser Wasserstoffperoxid zu erzeugen.

3. Sprühspender, der Folgendes umfasst:
ein Gehäuse, das einen Behälter zum Aufbewahren von Wasser, eine elektrolytische Zelle zum Aufnehmen von Wasser aus dem Behälter und eines sauerstoffhaltigen Gases und Erzeugen von Wasserstoffperoxid aus denselben und Mittel zum Umwälzen von Flüssigkeit zwischen dem Behälter und der elektrolytischen Zelle, um die Konzentration von Wasserstoffperoxid innerhalb des ausbewahrten Wassers zu steigern, enthält, und
eine Düse zum Spenden von wasserstoffperoxidhaltigem Wasser aus dem Behälter.

4. Sprühspender nach Anspruch 2 oder Anspruch 3, wobei die elektrolytische Zelle eine Gasdiffusionskathode, eine Membran und eine Anode umfasst, wobei der Sprühspender Mittel zum Zuführen eines sauerstoffhaltigen Gases zu einer Seite der Kathode umfasst und wobei die Mittel zum Umwälzen von Fluid dafür konfiguriert sind, Wasser zwischen der anderen Seite der Kathode und der Membran zu befördern.

5. Sprühspender nach Anspruch 4, wobei die Mittel zum Zuführen eines sauerstoffhaltigen Gases ein Gebläse umfassen, das Luft über die eine Seite der Kathode bläst.

6. Sprühspender nach Anspruch 4 oder Anspruch 5, wobei die Mittel zum Umwälzen von Fluid dafür konfiguriert sind, Wasser zwischen der Anode und der Membran zu befördern.

7. Sprühspender nach einem der Ansprüche 4 bis 6, wobei die Membran eine Protonenaustauschmembran umfasst.

8. Sprühspender nach einem der vorhergehenden Ansprüche, wobei das Gehäuse eine Basis und einen Korpus, der sich von der Basis aus nach oben erstreckt, definiert und wobei die elektrolytische Zelle in der Basis angeordnet ist.

9. Sprühspender nach einem der vorhergehenden Ansprüche, der ein Wasserenthärtungsmittel zum Aufnehmen von Wasser aus dem Behälter und Abgeben von enthärtetem Wasser an die elektrolytische Zelle umfasst.

10. Sprühspender nach einem der vorhergehenden Ansprüche, wobei das Wasserenthärtungsmittel einen Ionenaustausch-Werkstoff zum Entfernen von Übergangsmetallionen aus dem von dem Behälter empfangenen Wasser umfasst.

11. Sprühspender nach einem der Ansprüche 1 bis 8, der einen Ionenaustausch-Werkstoff zum Aufnehmen von Wasser aus dem Behälter, Entfernen von Übergangsmetallionen aus dem empfangenen Wasser und Abgeben von an Übergangsmetallionen abgereichertem Wasser an die elektrolytische Zelle umfasst.

12. Sprühspender nach einem der vorhergehenden Ansprüche, der eine Tülle umfasst, durch die Wasser in den Behälter eingeleitet wird, wobei die Tülle im Verhältnis zu dem Behälter zwischen einer offenen Stellung und einer geschlossenen Stellung bewegt werden kann.

13. Sprühspender nach einem der vorhergehenden Ansprüche, wobei das Gehäuse Steuerungsschaltungen zum Betreiben der elektrolytischen Zelle enthält.

14. Sprühspender nach Anspruch 12 und 13, wobei die Steuerungsschaltungen dafür konfiguriert sind, die Betriebsdauer der elektrolytischen Zelle in Abhängigkeit von der seit der Bewegung der Tülle verstrichenen Zeit zu steuern.

15. Sprühspender nach Anspruch 14, der einen Sensor zum Erkennen einer Bewegung der Tülle und zum Ausgeben eines Signals, das dieselbe anzeigt, an die Steuerungsschaltungen umfasst.

16. Sprühspender nach einem der Ansprüche 14 bis 15, wobei die Steuerungsschaltungen dafür konfiguriert sind, den Betrieb der elektrolytischen Zelle in Abhängigkeit von der Menge an Flüssigkeit, die seit der Bewegung der Tülle gespendet worden ist, zu steuern.

17. Sprühspender nach einem der vorhergehenden Ansprüche, wobei das Mittel zum Umwälzen von Flüssigkeit eine motorisierte Pumpe umfasst.

18. Sprühspender nach einem der vorhergehenden Ansprüche, in der Form eines in der Hand zu haltenden Sprühspenders.

19. Vorrichtung, die einen Sprühspender nach einem der vorhergehenden Ansprüche und eine Basisstation zum Aufnehmen des Sprühspenders und zum Zuführen von elektrischer Leistung zu der elektrolytischen Zelle umfasst.

20. Vorrichtung nach Anspruch 19, wobei der Sprühspender eine Batterie und ein Batterieladegerät zum Aufnehmen von elektrischer Leistung von der Basisstation umfasst.

## Revendications

1. Pulvérisateur, comprenant:
un boîtier, contenant un réservoir pour stocker un liquide, une cellule électrolytique pour recevoir le liquide du réservoir et pour accroître le niveau des propriétés oxydantes dans le liquide, et un moyen pour faire circuler le fluide entre le réservoir et la cellule électrolytique; et
une buse pour distribuer le liquide à partir du réservoir.

2. Pulvérisateur selon la revendication 1, dans lequel la cellule électrolytique est agencée de sorte à produire du peroxyde d'hydrogène à partir de l'eau reçue du réservoir.

3. Pulvérisateur, comprenant
un boîtier, contenant un réservoir pour stocker de l'eau, une cellule électrolytique pour recevoir l'eau provenant du réservoir et un gaz à base d'oxygène, et produisant du peroxyde d'hydrogène à partir de celui-ci, et un moyen pour faire circuler le fluide entre le réservoir et la cellule électrolytique, pour accroître la concentration du peroxyde d'hydrogène dans l'eau stockée ; et
une buse pour distribuer l'eau à base de peroxyde d'hydrogène provenant du réservoir.

4. Pulvérisateur selon les revendications 2 ou 3, dans lequel la cellule électrolytique comprend une cathode à diffusion de gaz, une membrane et une anode, le pulvérisateur comprenant un moyen pour amener un gaz à base d'oxygène vers un côté de la cathode, le moyen de circulation du fluide étant destiné à transférer l'eau entre l'autre côté de la cathode et la membrane.

5. Pulvérisateur selon la revendication 4, dans lequel le moyen d'amenée d'un gaz à base d'oxygène comprend un ventilateur pour souffler de l'air au-dessus dudit un côté de la cathode.

6. Pulvérisateur selon les revendications 4 ou 5, dans lequel le moyen de circulation du fluide est destiné à transférer l'eau entre l'anode et la membrane.

7. Pulvérisateur selon l'une quelconque des revendications 4 à 6, dans lequel la membrane comprend une membrane échangeuse de protons.

8. Pulvérisateur selon l'une quelconque des revendications précédentes, dans lequel le boîtier définit une base et un corps, s'étendant vers le haut à partir de la base, la cellule électrolytique étant agencée dans la base.

9. Pulvérisateur selon l'une des revendications précédentes, comprenant un adoucisseur d'eau pour recevoir l'eau provenant du réservoir et transférer l'eau adoucie vers la cellule électrolytique.

10. Pulvérisateur selon l'une des revendications précédentes, dans lequel l'adoucisseur d'eau comprend une matière échangeuse d'ions pour éliminer les ions métalliques de transition de l'eau provenant du réservoir.

11. Pulvérisateur selon l'une des revendications 1 à 8, comprenant une matière échangeuse d'ions pour recevoir l'eau à partir du réservoir, éliminer les ions métalliques de transition de l'eau reçue, et transférer l'eau appauvrie en ions métalliques de transition vers la cellule électrolytique.

12. Pulvérisateur selon l'une quelconque des revendications précédentes, comprenant un bec à travers lequel l'eau est introduite dans le réservoir, le bec pouvant être déplacé par rapport au réservoir entre une position ouverte et une position fermée.

13. Pulvérisateur selon l'une quelconque des revendications précédentes, dans lequel le boîtier contient un circuit de commande pour actionner la cellule électrolytique.

14. Pulvérisateur selon les revendications 12 et 13, dans lequel le circuit de commande est destiné à contrôler la durée du fonctionnement de la cellule électrolytique sur la base du temps écoulé depuis le déplacement du bec.

15. Pulvérisateur selon la revendication 14, comprenant un capteur pour détecter le déplacement du bec et pour transmettre un signal indicatif de ce déplacement vers le circuit de commande.

16. Pulvérisateur selon l'une quelconque des revendications 14 à 15, dans lequel le circuit de commande est destiné à contrôler le fonctionnement de la cellule électrolytique sur la base de la quantité de liquide distribuée depuis le déplacement du bec.

17. Pulvérisateur selon l'une quelconque des revendications précédentes, dans lequel le moyen de circulation du fluide comprend une pompe à moteur.

18. Pulvérisateur selon l'une quelconque des revendications précédentes, sous forme d'un pulvérisateur portatif.

19. Appareil comprenant un pulvérisateur selon l'une quelconque des revendications précédentes, et une station de base pour recevoir le pulvérisateur et pour alimenter la cellule électrolytique en énergie électrique.

20. Appareil selon la revendication 19, dans lequel le pulvérisateur comprend une batterie et un chargeur de batterie pour recevoir l'énergie électrique à partir de la station de base.
